# FASCICULE DE BREVET EUROPEEN

(11) **EP 2 464 335 B1**
(45) Date de publication et mention de la délivrance du brevet: **06.01.2016**
(21) Numéro de dépôt: 10740690.2
(22) Date de dépôt: 11.08.2010
(51) Int. Cl.: A61P 9/00, A61K 9/107, A61K 9/127, A61K 31/232, A61K 31/455, A61K 47/14, A61K 47/24, A61K 9/00

(54) **COMPOSITION PHARMACEUTIQUE COMPRENANT UN ESTER DE DHA DESTINEE A ETRE ADMINISTREE PAR VOIE PARENTERALE**
PHARMAZEUTISCHE ZUSAMMENSETZUNG MIT EINEM DHA-ESTER ZUR PARENTERALEN VERABREICHUNG
PHARMACEUTICAL COMPOSITION INCLUDING A DHA ESTER TO BE PARENTERALLY ADMINISTERED

(30) Priorité: 11.08.2009 FR 0955612
(43) Date de publication de la demande: 20.06.2012
(73) Titulaire: Pierre Fabre Médicament, 92100 Boulogne-Billancourt (FR)
(72) Inventeur: LEVERD, Elie, F-81100 Castres (FR); VAN HOOGEVEST, Peter, CH-4416 Bubendorf (CH); KUNG, Elsa, CH-4052 Basel (CH); LEIGH, Mathew, CH-4132 Muttenz (CH)
(74) Mandataire: Regimbeau
(86) Numéro de dépôt international: PCT/EP2010/061701
(87) Numéro de publication internationale: WO 2011/018480

(56) Documents cités:
- WO-A1-01/64328
- WO-A1-2004/032910
- WO-A2-00/78795
- WO-A2-2007/147899
- US-A- 5 760 081
- JUMAA M ET AL: "Parenteral emulsions stabilized with a mixture of phospholipids and PEG-660-12-hydroxy-stearate: evaluation of accelerated and long-term stability" EUROPEAN JOURNAL OF PHARMACEUTICS AND BIOPHARMACEUTICS, ELSEVIER SCIENCE PUBLISHERS B.V., AMSTERDAM, NL LNKD- DOI:10.1016/S0939-6411(02)00057-7, vol. 54, no. 2, 1 septembre 2002 (2002-09-01), pages 207-212, XP004377365 ISSN: 0939-6411

## Description

La présente invention concerne une composition pharmaceutique pour administration parentérale comprenant un ester de l'acide docosahexaenoique.

Les acides gras oméga-3 sont des acides gras polyinsaturés présents notamment dans les algues, les poissons gras (saumon, maquereau, sardine, thon), le colza, la noix, le soja...En règle générale, la classification des acides gras CH₃-(CH₂)ₙ-COOH est basée sur la longueur de la chaîne carbonée (courte pour n = 2 à 4, moyenne pour n = 6 à 8 et longue pour n égal ou > 10), le nombre de doubles liaisons (insaturé, mono ou polyinsaturé) et la position des doubles liaisons en partant du carbone du groupe carboxyle. Le système de référence ω indique quant à lui la longueur de la chaîne carbonée, le nombre de doubles liaisons et la position de la double liaison la plus proche du carbone ω en partant de ce carbone ω qui est par définition le dernier carbone de la chaîne, le plus loin du groupe carboxyle.

Les principaux acides gras du groupe ω 3 sont :
- l'acide linolénique 18 : 3 (9, 12, 15) ou ALA
- l'acide eicosapentaenoïque 20 : 5 (5, 8, 11, 14, 17) ou EPA
- l'acide docosahexaenoïque 22 : 6 (4, 7, 10, 13, 16, 19) ou DHA

Les acides gras oméga-3 procurent de nombreux effets bénéfiques pour la santé humaine.

La demande de brevet WO 01/46115 A1 rappelle les bienfaits d'une alimentation à base d'huile de poisson, riche en EPA et en DHA, sur la diminution des accidents cardiovasculaires. Une forme parentérale d'EPA et de DHA y est mentionnée sous forme de suspension de particules globulaires obtenue par des méthodes bien connues de l'homme du métier par exemple en diluant un tensioactif détergent non ionique dans de l'eau, en le chauffant puis en ajoutant l'ester de DHA et d'EPA. Cette demande décrit également des émulsions destinées à une administration intraveineuse contenant de l'huile de soja et des triglycérides via l'évocation d'une publication page 8 ligne 2 (Billman et al. 1997 Lipids 32 1161-1168).
Toutefois, il est difficile d'obtenir par un tel procédé des formulations parentérales stables prêtes à l'emploi, ou à reconstituer à partir de la forme lyophilisée, dont la taille moyenne des particules est inférieure à 100 nm, permettant l'incorporation d'une grande quantité de principe actif et ne contenant pas une trop forte proportion de tensioactif qui peut à terme se révéler toxique.

Parmi les esters de DHA, l'ester nicotinique ou pyridin-3-ylmethyl-cis-4,7,10,13,16,19-acide docosahexaenoïque est un ester très intéressant au niveau thérapeutique.

La formule développée de l'ester nicotinique de DHA ou pyridin-3-ylmethyl-cis-4,7,10,13,16,19-acide docosahexaenoïque est donnée ci-dessous :

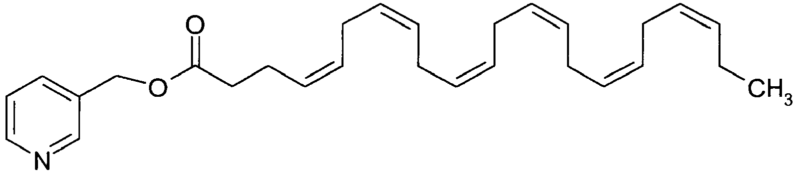

La masse moléculaire est 419,60 g, correspondant à C₂₈ H₃₇ NO₂.

C'est un composé très lipophile (Log P voisin de 7) dont la solubilité à l'équilibre dans l'eau est < 1 µg/ml. Il est donc particulièrement difficile à formuler pour une administration parentérale.

Un autre ester de DHA particulièrement intéressant est l'ester panthénique de DHA, autrement appelé docosahexaénéoate de panthényle, en particulier le monoester panthénique de DHA 2,4-dihydroxy-3,3-diméthylbutanamido) propyl docosa 4,7, 10, 13, 16, 19- hexanoate, ayant la formule A suivante : 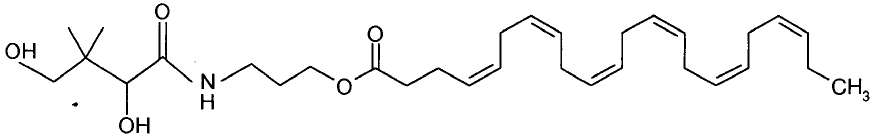 ou l'un de ses sels pharmaceutiquement acceptables, énantiomères, diastéréoisomères, ou leur mélange, y compris les mélanges racémiques.

Plus particulièrement, l'ester peut être le monoester panthénique de DHA de formule B suivante : 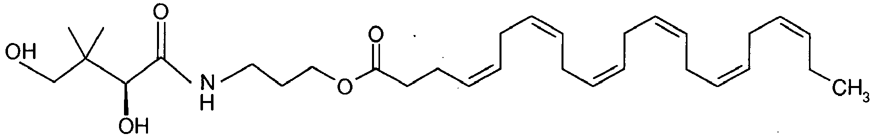 autrement appelé « ester DHA de D-panthénol ».

Ces esters sont très efficaces par exemple dans le traitement de la fibrillation auriculaire comme indiqué dans WO 2007/147899. Ils agissent en tant que prodrogue, libérant le DHA dans l'organisme, après hydrolyse.

Dans la présente invention, on entend désigner par « énantiomères » des composés isomères optiques qui ont des formules moléculaires identiques mais qui diffèrent par leur configuration spatiale et qui sont des images dans un miroir non superposables. On entend par « diastéréoisomères » les isomères optiques qui ne sont pas des images dans un miroir l'un de l'autre. Au sens de la présente invention, un « mélange racémique » est un mélange en proportions égales des énantiomères lévogyre et dextrogyre d'une molécule chirale.

Dans la présente invention, on entend désigner par « pharmaceutiquement acceptable » ou « acceptable sur le plan pharmaceutique » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sels pharmaceutiquement acceptables » d'un composé des sels qui sont pharmaceutiquement acceptables, comme défini ici, et qui possèdent l'activité pharmacologique souhaitée du composé parent. De tels sels comprennent :
(1) les sels d'addition d'acide formés avec des acides minéraux tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Les sels pharmaceutiquement acceptables préférés sont les sels formés à partir d'acide chlorhydrique, d'acide trifluoroacétique, d'acide dibenzoyl-L-tartrique et d'acide phosphorique.

Il devrait être compris que toutes les références aux sels pharmaceutiquement acceptables comprennent les formes d'addition de solvants (solvates) ou les formes cristallines (polymorphes) tels que définis ici, du même sel d'addition d'acide.

Les inventeurs ont découvert de façon surprenante qu'il était possible de préparer des compositions comprenant un ester de DHA et destinées à une administration par voie parentérale en utilisant une association de deux types de tensio-actifs, un ester d'acide gras polyoxyéthyléné et un dérivé phospholipidique.

La présente invention concerne donc une composition pharmaceutique destinée à être administrée par voie parentérale, comprenant des particules submicroniques d'ester de l'acide docosahexaenoïque, dispersées dans une phase aqueuse à l'aide d'un mélange d'au moins deux tensioactifs choisis parmi a) au moins un ester d'acide gras polyoxyéthyléné et b) au moins un dérivé phospholipidique.

Selon un mode de réalisation de l'invention, la composition ne contient comme tensioactif que les deux types de tensio-actifs a) et b).

L'ester de l'acide docosahexaenoïque peut être de toute sorte. Il peut en particulier s'agir d'un ester éthylique ou d'un ester avec une vitamine B tel que décrit dans la demande de brevet WO 2007/147899.

Dans un mode de réalisation de l'invention, il s'agit de l'ester nicotinique de DHA, c'est-à-dire le pyridin-3-ylmethyl-cis-4,7,10,13,16,19-acide docosahexaenoïque, ou de l'ester panthénique de DHA, en particulier le monoester panthénique de DHA 2,4-dihydroxy-3,3-diméthylbutanamido) propyl docosa 4,7, 10, 13, 16, 19- hexanoate, et plus particulièrement l'ester DHA de D-panthénol, qui sont particulièrement difficiles à formuler pour une administration par voie parentérale.

Dans un mode de réalisation de la présente invention, la concentration en ester de l'acide docosahexaenoïque est supérieure ou égale à 10 mg/ml, par exemple, supérieure ou égale à 30 mg/ml.

Le premier agent tensio-actif (a) appartient au groupe des esters d'acides gras polyoxyéthylénés. Les esters d'acides gras polyoxyéthylénés peuvent être obtenus par réaction entre un acide gras et un oxyde d'éthylène ou un polyéthylène glycol.

En particulier, les esters d'acides gras polyoxyéthylénés selon l'invention ont la formule suivante (1) ou (2):
(1) RCOO.(O.CH₂CH₂)ₙH
(2) R₁COO.(O.CH₂CH₂)ₙCOOR₂
dans lesquels R, R₁ et R₂ représentent indépendamment l'un de l'autre le groupe alkyle ou alcényle de l'acide gras parent et n représente la longueur de la chaîne polymérique, en motifs oxyéthylène. n est par exemple, entre 10 et 60, par exemple entre 12 et 20, par exemple 15.

L'acide gras peut-être saturé ou insaturé. Il est hydroxylé. Les acides gras saturés les plus communs sont comme suit:

| Nom commun | Nom IUPAC | Structure chimique | Abbr. | Point de fusion (°C) |
|---|---|---|---|---|
| Butyrique | acide butanoïque | CH₃(CH₂)₂COOH | C4:0 | -8 |
| Caproïque | acide héxanoique | CH₃(CH₂)₄COOH | C6:0 | -3 |
| Caprylique | acide octanoique | CH₃(CH₂)₆COOH | C8:0 | 16-17 |
| Caprique | acide décanoique | CH₃(CH₂)₈COOH | C10:0 | 31 |
| Laurique | acide dodécanoique | CH₃(CH₂)₁₀COOH | C12:0 | 44-46 |
| Myristique | acide tétradecanoique | CH₃(CH₂)₁₂COOH | C14:0 | 58.8 |
| Palmitique | acide hexadécanoique | CH₃(CH₂)₁₄COOH | C16:0 | 63-64 |
| Stéarique | acide octadécanoique | CH₃(CH₂)₁₆COOH | C18:0 | 69.9 |
| Arachidique | acide éicosanoique | CH₃(CH₂)₁₈COOH | C20:0 | 75,5 |
| Béhénique | acide docosanoique | CH₃(CH₂)₂₀COOH | C22:0 | 74-78 |
| Lignocérique | acide Tétracosanoique | CH₃(CH₂)₂₂COOH | C24:0 | |

En conséquence, R, R₁ ou R₂ est , par exemple, une chaîne alkyle ou alcényle, p. ex. alkyle, linéaire ou ramifiée, par exemple linéaire, en C₃-C₂₃, par exemple en C₁₁-C₂₃, par exemple en C₁₅-C₂₁, par exemple, l'acide gras est un acide gras saturé à longue chaine, i.e. ayant plus de 16 atomes de carbone.

Dans un mode de réalisation l'acide gras a entre 12 et 24 atomes de carbone, par exemple, entre 14 et 22 atomes de carbone, par exemple entre 16 et 20 atomes de carbone, par exemple, il a 18 atomes de carbone, par exemple il s'agit de l'acide stéarique.

Il peut s'agir de mono ou de diester d'acides gras, ou d'un mélange de ceux-ci. Dans un mode de réalisation de l'invention, il s'agit d'un mélange de mono et di ester d'acide gras. Par exemple, l'ester d'acide gras polyoxyéthyléné est le macrogol-15 hydroxystéarate (dénomination commerciale: SOLUTOL HS15, fabricant: BASF, Ludwigshafen, Allemagne). C'est un agent solubilisant non ionique, constitué principalement de monoesters et de diesters de l'acide 12-hydroxystéarique et de macrogols obtenu par éthoxylation de l'acide 12-hydroxystéarique. Le nombre de moles d'oxyde d'éthylène ayant réagi par mole d'acide 12-hydroxystéarique est de 15. Il se présente sous forme d'une masse cireuse jaunâtre, très soluble dans l'eau.

En particulier les esters d'acides gras polyoxyéthylénés utilisables dans le cadre de la présente invention sont des tensioactifs non ioniques.

Le deuxième agent tensio-actif (b) appartient au groupe des dérivés phospholipidiques: il peut ainsi s'agir de lécithines d'origine naturelle, comme par exemple les lécithines de soja ou d'oeuf, de phospholipides d'origine naturelle, comme par exemple les phospholipides de soja ou d'oeuf, ou de phospholipides synthétiques, ou leur mélange. Il peut par exemple s'agir de phospholipides neutres tels que les phosphatidylcholines, comme par exemple le 1,2-dimyristoylphosphatidylcholine ou le 1-palmitoyl-2-oleoylphosphatidylcholine, et les phosphatidylethanolamines, par exemple les phosphatidylcholines ,de phospholipides chargés négativement tels que les phosphatidylglycerols, comme par exemple le 1,2-dimyristoylphosphatidylglycerol, les phosphatidylserines, les phosphatidylinositols et les acides phosphatidiques, p. ex. les phosphatidylglycerols, ou de leurs mélanges. Par exemple, il s'agit d'un phospholipide neutre tel qu'une phosphatidylcholine, ou il peut s'agir d'un mélange d'un phospholipide neutre et d'un phospholipide chargé négativement, par exemple du 1-palmitoyl-2-oleoylphosphatidylcholine et du 1,2-dimyristoylphosphatidylglycerol.

Dans le mélange d'un phospholipide neutre et d'un phospholipide chargé négativement, le pourcentage massique en phospholipide chargé négativement par rapport à la composition totale du mélange est inférieur à 10 %, par exemple, compris entre 1 % et 5 %, p. ex. égal à 3 %.

Il peut également s'agir d'un mélange d'une lécithine d'origine naturelle avec un phospholipide chargé négativement, en particulier d'une lécithine d'oeuf avec le 1,2-dimyristoylphosphatidylglycerol.

Le ratio massique tensioactif de type a) / tensioactif de type b) peut être compris entre1/3 et 3/1 par exemple, il est de 1/1.

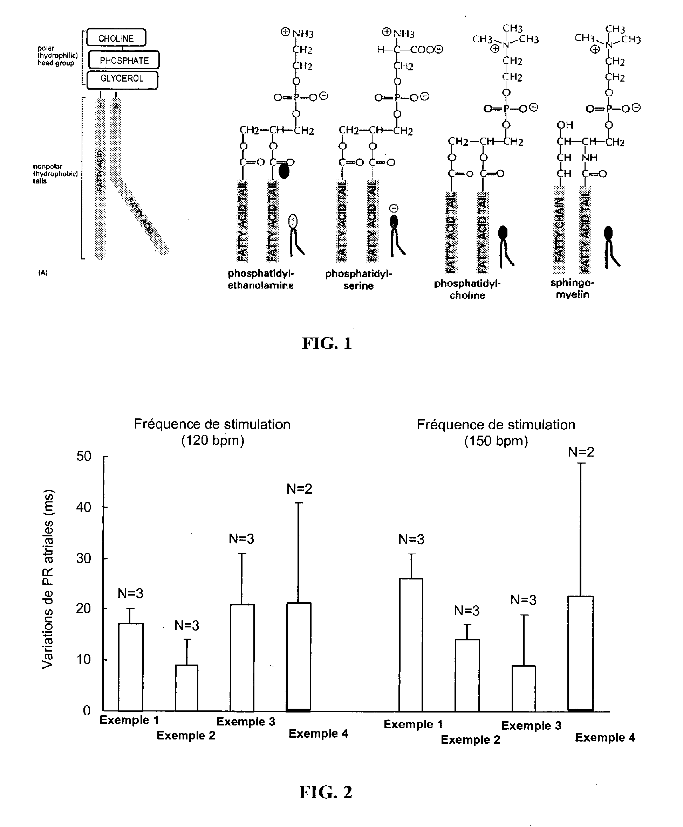

Les dérivés phospholipidiques ont la structure générale indiquée dans la figure 1.

Dans un mode de réalisation de l'invention, le dérivé phospholipidique ne contient pas de lécithine de soja ou de phospholipide de soja.

Dans un mode de réalisation les particules submicroniques sont des particules submicroniques telles que des micelles mixtes ou des vésicules ou des hybrides de structures micellaires et vésiculaires.

Une micelle mixte selon l'invention est une micelle, par exemple un agrégat, constituée par un mélange des deux types différents de tensioactifs (a) et (b), la tête polaire hydrophile des molécules de tensioactifs étant dirigée vers la phase aqueuse et la chaîne hydrophobe dirigée vers l'intérieur, en interaction avec l'ester de DHA.

Une vésicule selon l'invention est une structure dans laquelle les surfactants sont organisés suivant un arrangement en bi-couches identique à ce qui est présent dans les membranes cellulaires. Ces surfactants entourent une vacuole ou cavité aqueuse.
Un hybride de structure micellaire et vésiculaire selon l'invention est une structure intermédiaire entre micelle mixte et vésicule, avec ou non l'existence de cette vacuole.

Ainsi, par exemple, la composition selon l'invention est une dispersion de micelles mixtes ou de vésicules ou d'hybrides de structures micellaires et vésiculaires. Par exemple, ces particules montrent des faces de fracture plane sous observation en microscopie électronique après cryofracture.

Selon un mode de réalisation de la présente invention, la composition selon l'invention n'a pas la forme d'une émulsion.

Dans un mode de réalisation de la présente invention, les particules submicroniques ont une taille moyenne < 100 nm, par exemple comprise entre 25 et 70 nm, par exemple avec une polydispersité < 0,5, (la taille et la polydispersité sont déterminées par spectroscopie par corrélation de photons sur appareil Zetasizer de Malvern).

En plus des particules submicroniques décrites précédemment, la composition selon l'invention peut également contenir, de façon optionnelle:
- des agents anti-oxydants qui protègent l'ester de DHA, en particulier l'ester nicotinique ou l'ester panthénique de DHA, et en particulier l'ester DHA de D-panthénol, de l'oxydation par l'oxygène dissous dans la composition. Citons, à titre d'exemples non limitatifs: l'acide ascorbique et ses dérivés, les composés libérant du dioxyde de soufre comme le métabisulfite de sodium, le gallate de propyle, le butylhydroxytoluène, le butylhydroxyanisole, le D,L-α-tocophérol, les dérivés de l'acide éthylenediaminetétracétique et leurs combinaisons. Leur teneur est comprise entre 0,01% et 1% (m/V), plus précisément entre 0,01% et 0,50% (m/V). L'action des agents anti-oxydants est complétée de façon pertinente par la mise en oeuvre de gaz inertes comme l'azote ou l'argon durant la fabrication et le conditionnement des compositions injectables.
- des agents régulateurs de pH. Ils sont connus de l'homme de l'art et incluent des acides et des bases, minéraux ou organiques, ainsi que des systèmes tampons. Leur utilisation permet de fixer le pH de la composition selon l'invention à une valeur comprise entre pH = 4 et pH = 9, compatible avec une administration par voie parentérale. Il peut ainsi s'agir d'acide ascorbique.
- des agents permettant d'ajuster l'osmolarité de la composition selon l'invention afin d'en assurer l'isotonie avec le sang. Ceux-ci sont par exemple des molécules neutres comme les carbohydrates, p. ex. des monosaccharides réduits ou non [par exemple glucose ou mannitol à une concentration ≤ 5% (m/V)] ou des disaccharides [par exemple saccharose à une concentration ≤ 10% (m/V)].
- l'eau pour préparations injectables comme milieu dispersant, par exemple une solution de glucose à 5% ou une solution de chlorure de sodium à 0,9%.

Les compositions selon l'invention se présentent sous forme de dispersions aqueuses prêtes à être administrées ou sous forme de dispersions lyophilisées qui sont reconstituées extemporanément avec de l'eau pour préparations injectables avant administration.

A titre d'exemple non limitatif, pour une concentration de 30 mg/ml - soit 3% (m/V) - en ester nicotinique de DHA, les concentrations en SOLUTOL HS15 et en dérivé phospholipidique sont respectivement comprises entre 2,5 et 5% (m/V) et 0 et 10% (m/V). Préférentiellement, une composition avec 5% (m/V) de SOLUTOL HS15 et 5% (m/V) de dérivé phospholipidique sera utilisée.

Les formulations indiquées dans le tableau 1 ci-dessous illustrent la présente invention. Elles ont été préparées par le premier procédé décrit ci-après.

| | 1 | 2 | 3 | 4 |
|---|---|---|---|---|
| ester nicotinique de DHA | 3,00 g | 3,00 g | 3,00 g | 3,00 g |
| 1,2-Dimyristoylphosphatidylcholine | 5,00 g | | | |
| 1-Palmitoyl-2-oleoylphosphatidylcholine | | 4,85 g | 4,85 g | |
| 1,2-Dimyristoylphosphatidylglycero 1 | | 0,15 g | 0,15 g | 0,15 g |
| Lécithine d'oeuf | | | | 4,85 g |
| SOLUTOL HS 15 | 5,00 g | 5,00 g | 5,00 g | 5,00 g |
| Acide ascorbique | | | 0,20 g | |
| Solution de glucose à 5 % | qsp 100 ml | qsp 100ml | qsp 100 ml | qsp 100 ml |
| Taille moyenne des particules | 28,6 nm | 52,0 nm | 65,1 nm | 50,6 nm |
| Polydispersité | 0,4 | 0,3 | 0,1 | 0,2 |

La présente invention concerne en outre un procédé de préparation de la composition selon la présente invention qui comprend les étapes suivantes :
- disperser l'ester de DHA, en particulier l'ester nicotinique de DHA ou l'ester panthénique de DHA, en particulier le monoester panthénique de DHA de formule A ou de formule B, le(s) dérivé(s) phospholipidique(s) et l'ester d'acide gras polyoxyéthyléné, par exemple le SOLUTOL HS15, dans une solution aqueuse destinée à une administration parentérale, p. ex. une solution de glucose à 5%, sous agitation par exemple magnétique ou agitation à ancre, par exemple à environ 700 tours/min., jusqu'à obtention d'une dispersion homogène mais turbide,
- homogénéiser la dispersion obtenue, par exemple à l'aide d'une turbine rotor / stator, (par exemple du type Labortechnik T25 (IKA) ou équivalent), p. ex. à environ 13500 tours/min, puis d'un homogénéiseur haute pression, (par exemple du type EMULSIFLEX C-5 (AVESTIN) ou équivalent), p. ex. à une pression comprise entre 1300 et 1600 bars,
- stériliser la formulation colloïdale obtenue, par exemple par passage à travers un filtre stérilisant 0,2 µm tel que du type DURAPORE^{®} de MILLIPORE ou équivalent.

Le procédé peut en outre comprendre une étape supplémentaire consistant à répartir le filtrat stérile, en atmosphère aseptique, dans les articles de conditionnement primaire propres et préalablement stérilisés.

Ce conditionnement primaire est par exemple soit une ampoule scellée, soit un flacon fermé par un bouchon en élastomère et une capsule de sertissage, soit une seringue pré-remplie prête à l'administration.

Dans un procédé alternatif, les deux tensioactifs a) et b) peuvent être co-homogénéisés préalablement dans la solution aqueuse destinée à une administration parentérale telle qu'une solution de glucose à 5%, avant ajout de l'ester de DHA, en particulier l'ester nicotinique ou l'ester panthénique de DHA, en particulier le monoester panthénique de DHA de formule A ou de formule B et homogénéisation finale.

Dans un autre procédé alternatif, le tensioactif a) et l'ester de DHA sont homogénéisés préalablement à l'ajout du tensio-actif b) suivi de l' homogénéisation de l'ensemble. Un solvant est ensuite ajouté, par exemple, un mélange alcool/ eau, par exemple éthanol/eau, par exemple 15,8:1 v/v respectivement, et l'homogénéisation se poursuit. Le solvant est ensuite éliminé par séchage sous vide. Le complexe obtenu est alors dilué dans une solution de glucose à 5%. La dispersion obtenue est éventuellement homogénéisée, par exemple à l'aide d'un homogénéiseur à haute pression. La formulation obtenue est stérilisée par passage au travers d'un filtre 0,22µm, tel que décrit précédemment.

Les éventuels agents anti-oxydants, agents régulateurs de pH et/ou agents isotonisants sont dissous dans la phase aqueuse soit avant, soit après l'homogénéisation.

Dans un mode de réalisation, la composition pharmaceutique selon l'invention est destinée à être administrée par voie intraveineuse, par voie intra-artérielle, par voie intracardiaque, par voie sous-cutanée, par voie intradermique, par voie intramusculaire, par voie intra-rachidienne, par voie intrathécale, par voie intra-péritonéale, par voie intraoculaire, par voie intra-ventriculaire, par voie intra-péricardique, par voie intra-durale ou par voie intra-articulaire.

De façon générale, les compositions selon l'invention sont administrées par voie intraveineuse, telles quelles ou après dilution dans des solutions physiologiquement acceptables comme les solutions de glucose à 5% ou les solutions de chlorure de sodium à 0,9%.

Ces compositions peuvent aussi être administrées par voie intra-artérielle, par voie intracardiaque, par voie sous-cutanée, par voie intradermique, par voie intramusculaire ou par voie intra-rachidienne.

La présente invention concerne en outre une composition pharmaceutique selon l'invention, pour utilisation à titre de médicament.

Le médicament est destiné à la prévention et/ou au traitement des maladies cardiovasculaires, par exemple choisies parmi l'arythmie supraventriculaire et/ou ventriculaire, la tachycardie et/ou la fibrillation, p. ex. la fibrillation atriale ; à la prévention et/ou au traitement de maladies représentées par des défauts de la conduction électrique des cellules du myocarde ; à la prévention et/ou au traitement de facteurs de risques multiples de maladies cardiovasculaires, par exemple choisis parmi l'hypertriglycéridémie, l'hypercholestérolémie, les hyperlipidémies, les dyslipidémies, p. ex. les dyslipidémies mixtes, thrombose artérielle et veineuse provoquée par l'hyperactivité de facteur II (thrombine) de coagulation sanguine et/ou agrégation plaquettaire, et/ou l'hypertension artérielle; à la prévention primaire ou secondaire et/ou au traitement de maladies cardiovasculaires dérivant de l'arythmie supraventriculaire et/ou ventriculaire, de la tachycardie, de la fibrillation et/ou de défauts de la conduction électrique induits par l'infarctus du myocarde, avantageusement de la mort subite ; et/ou au traitement post-infarctus.

L'invention sera mieux comprise à la lumière des figures et des tests sur la fibrillation atriale qui suivent :
La figure 1 représente la structure générale des dérivés phospho lipidiques.
La figure 2 représente la mesure des périodes réfractaires atriales chez les porcs anesthésiés lors de l'administration des compositions selon l'invention (bolus 10mg/kg d'ester nicotinique de DHA + perfusion 10mg/kg d'ester nicotinique de DHA sur 40 minutes) avec une fréquence de stimulation de 120 bpm et de 150bpm..

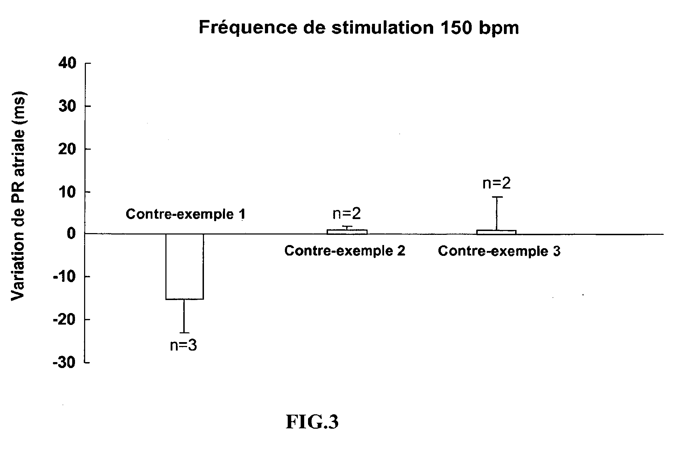

La figure 3 représente la mesure des périodes réfractaires atriales chez les porcs anesthésiés lors de l'administration de compositions comparatives (contre-exemples) (bolus 10mg/kg d'ester nicotinique de DHA + perfusion 10mg/kg d'ester nicotinique de DHA sur 40 minutes) avec une fréquence de stimulation de 150 bpm.

### Exemple 1 :

A titre d'exemple, l'activité des compositions selon l'invention indiquées dans le tableau 1 dans le traitement de la fibrillation atriale est parfaitement mise en évidence par le test de pharmacologie décrit ci-après.

Des porcs Landrace mâles (22-25 kg) sont anesthésiés à l'isoflurane (1,5-3%). Les animaux sont ensuite intubés et ventilés afin de garder les valeurs de gaz artériel dans des limites physiologiques. Une thoracotomie gauche est effectuée au niveau du quatrième espace intercostal. L'artère et la veine mammaire sont isolées, des cathéters sont insérés dans la veine pour l'administration des produits à tester et dans l'artère pour mesurer la pression artérielle et recueillir des prélèvements de sang. Un lit péricardique est pratiqué. Un électrocardiogramme (ECG) atrial est enregistré en continu, trois électrodes sont placées sur l'épicarde et suturées. De ce fait, l'ECG nous renseigne uniquement sur l'étage atrial. Deux électrodes bipolaires sont également placées sur l'oreillette gauche: elles permettront de stimuler électriquement l'oreillette à une fréquence donnée.

### Protocole expérimental :

Après une période de récupération suffisante, la détermination de la période réfractaire atriale sous condition contrôle peut commencer. Un train continu de stimuli (S1) est lancé à un voltage très faible (0,1 V), insuffisant pour entraîner le coeur.

Progressivement le voltage est augmenté (par pas de 0,1 V) pour trouver le seuil de stimulation qui permet de suivre la fréquence imposée. La recherche de ce seuil est réalisée à chaque fréquence de stimulation. Il y a 4 fréquences de stimulation, 90, 120, 150 et 180 bpm. Si le porc a une fréquence cardiaque de base supérieure à 90 bpm la première fréquence de stimulation n'est pas réalisée. De même, si la fréquence de base est faible (< 90 bpm) la dernière fréquence de stimulation (180 bpm) testée n'entraîne pas toujours le coeur. Une fois le seuil trouvé, le voltage de la stimulation S1 (train de 10 stimuli) est égal à 2 fois le seuil, et le voltage de l'extrastimulus S2 est égal à 4 fois le seuil. Tous les 10 S1, un extrastimulus S2 est déclenché pendant la période réfractaire (c'est à dire 80 ms après le dernier S1, la période réfractaire attendue est d'au moins 100 ms), puis tous les 10 stimuli S1, l'extrastimulus est écarté du dernier S1 (incrément de 5 ms en 5 ms) jusqu'à ce qu'il enclenche lui même un battement. L'intervalle le plus long n'entraînant pas de réponse à S2 est la période réfractaire atriale. La période réfractaire est évaluée 3 fois de suite (expression de la moyenne), à 4 fréquences de stimulation différentes soit 12 mesures. Le produit à tester est administré sous forme de bolus puis de perfusion sur 40 min (temps nécessaire pour évaluer toutes les périodes réfractaires). Les seuils ne sont pas recalculés, les périodes réfractaires sont tout de suite mesurées (5 min après la fin du bolus). Si le produit est actif les périodes réfractaires doivent être augmentées par rapport à la phase contrôle.

Il est remarquable de constater que seules les compositions de l'invention répondent positivement à ce test pharmacologique comme l'illustrent les figures 2 et 3.

Les contre-exemples sont des formulations très proches des formulations selon l'invention comme indiquées dans le tableau 2 ci-dessous mais elles démontrent une activité pharmacologique inférieure ou négative.

**Tableau 2**

| | **Contre-exemple 1** | **Contre-exemple 2** | **Contre-exemple 3** |
|---|---|---|---|
| ester nicotinique de DHA | 3,00 g | 3,00 g | 3,00 g |
| 1,2-Dimyristoylphosphatidylcholine | | | 2,00 g |
| SOLUTOL HS 15 | 5,00 g | | |
| Polysorbate 80 | | 2,50 g | |
| Triglycérides à chaîne moyenne | | 2,50 g | |
| 1,2-Dimyristoylphosphatidylglycerol | | | 0,50 g |
| Solution de glucose à 5 % | qsp 100 ml | qsp 100 ml | qsp 100 ml |

On remarque donc que l'absence d'un des tensioactifs ((a) ou (b) respectivement contre-exemple 3 et contre-exemple 1) dans la composition destinée à l'administration parentérale contenant l'ester nicotinique de DHA ou l'utilisation d'un autre tensioactif que le a) ou b) (contre exemple 2) ne permet pas d'obtenir l'activité désirée.

### Exemple 2 : Formulation injectable comprenant l'ester panthénique de DHA de formule B

POPC = 1-palmitoyl-2-oleoyl phosphatidylcholine
EPCS = phosphatidyl choline d'oeuf
DMPG = 1 ,2, dimyristoylphosphatidylglycerol

### 1- Procédé de préparation par complexation en présence de solvant :

Le solutol HS 15 et l'ester panthénique de DHA de formule B sont mélangés à une température de 50°C jusqu'à homogénéisation. Les phospholipides sont ensuite ajoutés et le mélange est placé sous agitation magnétique à une température de 50°C pour une durée de 1 h à 1h30 sous atmosphère inerte par exemple, sous nitrogène. L'éthanol et l'eau, par exemple, éthanol/eau [15,8 :1 v/v], sont ajoutés au mélange et l'agitation est poursuivie sous atmosphère inerte jusqu'à ce que les lipides soient complètement dispersés. Le mélange est alors traité pendant 30 min par ultrasonication pour obtenir la dispersion complète des phospholipides. La dispersion résultante est placée sous vide pendant au moins 24 h afin d'éliminer le solvant et l'eau.

Le complexe est dilué dans une solution de glucose à 5 %. Si la dispersion est opaque, elle est homogénéisée à l'homogénéiseur à haute pression jusqu'à ce que la dispersion soit translucide, claire. Elle est stérilisée sur filtre PVDF 0.22 µm.

### 2- Procédé de préparation par mélange d'ester panthénique de DHA avec une dispersion de phospholipides et de Solutol HS 15.

Les phospholipides, le Solutol HS 15 et la solution de glucose à 5% sont pesés et le mélange est agité magnétiquement et chauffé à 60°C jusqu'à ce qu'une dispersion soit obtenue. La dispersion est ensuite traitée à l'homogénéiseur à haute pression en cycle jusqu'à ce qu'une dispersion, claire et/ ou translucide soit obtenue.

L'ester panthénique de DHA de formule B est ensuite ajouté à ladite dispersion et le mélange est homogéniésé pendant 1 à 2 minutes à 13500 rev/min. La dispersion est traitée à l'homogénéiseur à haute pression en cycle jusqu'à ce qu'une dispersion, claire et/ ou translucide soit obtenue, par exemple un maximum de 5 cycles. La dispersion est filtrée à travers in filtre PVDF de 0.22 µm.

### 3- Procédé de préparation par mélange de l'ester panthénique de DHA, des phospholipides et du Solutol HS 15.

Tous les composants sont pesés dans un flacon, un barreau aimanté est ajouté et le flacon est scellé sous atmosphère inerte par exemple sous azote. Le mélange est agité pendant 30 min ou jusqu'à ce qu'une dispersion homogène soit obtenue. La dispersion est homogénéisée à 13500 rev/min pendant 1 à 2 minutes et traitée par un homogénéiseur à haute pression jusqu'à ce qu'une dispersion translucide et/ ou claire ou qu'une transmission constante soit obtenue, par exemple après 6 cycles. Les formulations sont filtrées sur filtres à 0.22µm et l'aspect visuel, l'apparence au microscope, le pH, la transmission et la taille des particules est déterminée à T0 et après stockage pendant 2 semaines à 4°C, 25°C et 40°C.

Le développement de la formulation a été réalisé avec les compositions suivantes indiquées dans le tableau 3 ci-dessous :

**Tableau 3**

| **Composés** | 30 mg P-DHA/ml POPC 30 formulation | | mg P-DHA/ml EPCS formulation | |
|---|---|---|---|---|
| | poids | Concentration (mg/ml) | poids | Concentration (mg/ml) |
| Ester panthénique de DHA de formule B | 3,00 | 30,1 | 3,00 | 30,1 |
| Solutol HS 15 | 5,00 | 49,8 | 5 ,00 | 49,8 |
| POPC | 4,85 | 48,5 | - | - |
| EPC S | - | - | 48,5 | 48,5 |
| DMPG | 0,15 | 1,6 | 0,15 | 1,6 |
| 5 % glucose solution | 87,00 | 870,1 | 87,00 | 870,1 |
| Total | 100 | 1000,1 | 100 | 1000,1 |

Les exemples ci-dessus ont permis d'évaluer si l'ester panthénique de DHA, par exemple l'ester panthénique de formule B, peut être combiné directement avec les excipients pour former un complexe qui peut ensuite être dilué avec une solution de glucose à 5% pour donner une dispersion convenant à l'injection.

La composition obtenue selon chacun des 3 procédés de préparation est analysée.

L'aspect visuel et l'apparence de la formulation sont observés au microscope, le pH, la transmission et la taille des particules est mesurée à T0 et après stockage de 2 à 4 semaines à 4, 25 et 40°C.

La conclusion issue de ces observations est que des compositions contenant 30 mg d'ester panthénique de DHA de formule B par ml de Solutol HS 15 et de POPC ou de EPCS avec DMPG et une solution de glucose à 5% peuvent être préparées selon ces trois procédés décrits. Le pH est resté stable sur les deux semaines, la taille des particules est restée inférieure à 100 nm. Aucun pic de dégradation n'a été observé sur les chromatogrammes HPLC.

## Revendications

1. Composition pharmaceutique destinée à être administrée par voie parentérale, comprenant des particules submicroniques d'ester de l'acide docosahexaenoïque, dispersées dans une phase aqueuse à l'aide d'un mélange d'au moins deux tensio-actifs choisis parmi a) au moins un ester d'acide gras polyoxyéthyléné et b) au moins un phospholipide.

2. Composition pharmaceutique selon la revendication 1, **caractérisée en ce que** l'ester d'acide docosahexaenoïque est l'ester nicotinique.

3. Composition pharmaceutique selon la revendication 1, **caractérisée en ce que** l'ester d'acide docosahexaenoïque est l'ester panthénique de DHA de formule A suivante : 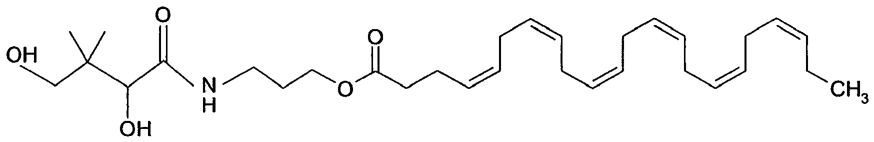 ou l'un de ses sels pharmaceutiquement acceptables, énantiomères, diastéréoisomères, ou leur mélange, y compris les mélanges racémiques.

4. Composition pharmaceutique selon la revendication 3, **caractérisée en ce que** l'ester d'acide docosahexaenoïque est l'ester panthénique de DHA de formule B suivante :

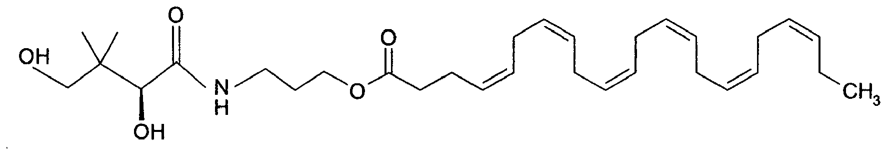

5. Composition pharmaceutique selon la revendication 1 à 4, **caractérisée en ce que** l'ester d'acide gras polyoxyéthyléné est le macrogol-15 hydroxystéarate.

6. Composition pharmaceutique selon l'une quelconque des revendications 1 à 5, **caractérisée en ce que** le phospholipide est choisi parmi les lécithines d'origine naturelle, comme par exemple les lécithines de soja ou d'oeuf, les phospholipides d'origine naturelle, comme par exemple les phospholipides de soja ou d'oeuf, les phospholipides synthétiques, ou leur mélange.

7. Composition pharmaceutique selon la revendication 6, **caractérisée en ce que** le dérivé phospholipidique est un mélange d'un phospholipide neutre et d'un phospholipide chargé négativement.

8. Composition pharmaceutique selon l'une quelconque des revendications 1 à 7, **caractérisée en ce que** la concentration en ester de l'acide docosahexaenoïque est supérieure ou égale à 10 mg/ml, avantageusement supérieure ou égale à 30 mg/ml.

9. Composition pharmaceutique selon l'une quelconque des revendications 1 à 8, **caractérisée en ce qu'**il s'agit d'une dispersion de micelles mixtes ou de vésicules ou d'hybrides de structures micellaires et vésiculaires.

10. Procédé de préparation d'une composition pharmaceutique selon l'une quelconque des revendications 1 à 9, **caractérisé en ce qu'**il comprend les étapes suivantes :
- disperser l'ester de DHA, en particulier l'ester nicotinique de DHA défini dans la revendication 2 ou l'ester panthénique de DHA défini aux revendications 3 ou 4, le(s) dérivé(s) phospholipidique(s) et l'ester d'acide gras polyoxyéthyléné, en particulier le macrogol 15 hydroxy stéarate, dans une solution aqueuse destinée à une administration parentérale, en particulier une solution de glucose à 5%, sous agitation, en particulier à environ 700 tours/min., jusqu'à obtention d'une dispersion homogène mais turbide,
- homogénéiser la dispersion obtenue, par exemple à l'aide d'une turbine rotor / stator, en particulier à environ 13500 tours/min, puis d'un homogénéiseur haute pression, en particulier à une pression comprise entre 1300 et 1600 bars,
- stériliser la formulation colloïdale obtenue par exemple par passage à travers un filtre stérilisant 0,2 µm.

11. Composition pharmaceutique selon l'une quelconque des revendications 1 à 9, **caractérisées en ce qu'**elle est destinée à être administrée par voie intraveineuse, par voie intra-artérielle, par voie intracardiaque, par voie sous-cutanée, par voie intradermique, par voie intramusculaire par voie intra-rachidienne, par voie intrathécale, par voie intra-péritonéale, par voie intraoculaire, par voie intra-ventriculaire, par voie intra-péricardique, par voie intra-durale ou par voie intra-articulaire.

12. Composition pharmaceutique selon l'une quelconque des revendications 1 à 9 ou 11, pour utilisation à titre de médicament.

13. Composition pharmaceutique pour utilisation à titre de médicament selon la revendication 12, **caractérisée en ce que** ledit médicament est destiné à la prévention et/ou au traitement des maladies cardiovasculaires, avantageusement choisies parmi l'arythmie supraventriculaire et/ou ventriculaire, la tachycardie et/ou la fibrillation, par exemple, la fibrillation atriale ; à la prévention et/ou au traitement de maladies représentées par des défauts de la conduction électrique des cellules du myocarde ; à la prévention et/ou au traitement de facteurs de risques multiples de maladies cardiovasculaires, par exemple choisis parmi l'hypertriglycéridémie, l'hypercholestérolémie, les hyperlipidémies, les dyslipidémies, avantageusement les dyslipidémies mixtes, thrombose artérielle et veineuse provoquée par l'hyperactivité de facteur II (thrombine) de coagulation sanguine et/ou agrégation plaquettaire, et/ou l'hypertension artérielle; à la prévention primaire ou secondaire et/ou au traitement de maladies cardiovasculaires dérivant de l'arythmie supraventriculaire et/ou ventriculaire, de la tachycardie, de la fibrillation et/ou de défauts de la conduction électrique induits par l'infarctus du myocarde, avantageusement de la mort subite ; et/ou au traitement post-infarctus.

## Patentansprüche

1. Pharmazeutische Zusammensetzung, die dafür vorgesehen ist, parenteral verabreicht zu werden, und die Docosahexaensäureester-Partikel im Submikronbereich umfasst, die in einer wässrigen Phase mithilfe einer Mischung von mindestens zwei Tensiden dispergiert sind, die aus a) mindestens einem Polyoxyethylenfettsäureester und b) mindestens einem Phospholipid ausgewählt sind.

2. Pharmazeutische Zusammensetzung nach Anspruch 1, **dadurch gekennzeichnet, dass** der Docosahexaensäureester der Nikotinsäureester ist.

3. Pharmazeutische Zusammensetzung nach Anspruch 1, **dadurch gekennzeichnet, dass** der Docosahexaensäureester der DHA-Panthenolester der folgenden Formel A: 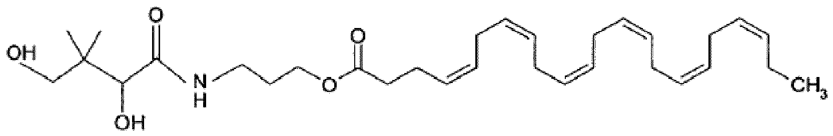 oder eines seiner pharmazeutisch akzeptablen Salze, Enantiomere, Diastereoisomere oder deren Mischung, einschließlich der racemischen Mischungen, ist.

4. Pharmazeutische Zusammensetzung nach Anspruch 3, **dadurch gekennzeichnet, dass** der Docosahexaensäureester der DHA-Panthenolester der folgenden Formel B ist:

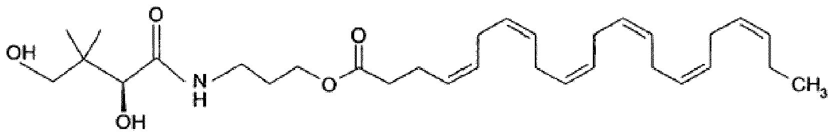

5. Pharmazeutische Zusammensetzung nach den Ansprüchen 1 bis 4, **dadurch gekennzeichnet, dass** der Polyoxyethylenfettsäureester das Macrogol-15-Hydroxystearat ist.

6. Pharmazeutische Zusammensetzung nach einem der Ansprüche 1 bis 5, **dadurch gekennzeichnet, dass** das Phospholipid aus den Lecithinen natürlichen Ursprungs, wie beispielsweise den Soja- oder Eilecithinen, den Phospholipiden natürlichen Ursprungs, wie beispielsweise den Soja- oder Eiphospholipiden, den synthetischen Phospholipiden oder deren Mischung ausgewählt ist.

7. Pharmazeutische Zusammensetzung nach Anspruch 6, **dadurch gekennzeichnet, dass** das Phospholipid-Derivat eine Mischung von einem neutralen Phospholipid und einem negativ geladenen Phospholipid ist.

8. Pharmazeutische Zusammensetzung nach einem der Ansprüche 1 bis 7, **dadurch gekennzeichnet, dass** die Konzentration des Docosahexaensäureesters größer gleich 10 mg/ml, vorteilhafterweise größer gleich 30 mg/ml ist.

9. Pharmazeutische Zusammensetzung nach einem der Ansprüche 1 bis 8, **dadurch gekennzeichnet, dass** es sich um eine Dispersion von gemischten Mizellen oder Vesikeln oder Hybriden mit Mizellen- und Vesikelstrukturen handelt.

10. Verfahren zur Herstellung einer pharmazeutischen Zusammensetzung nach einem der Ansprüche 1 bis 9, **dadurch gekennzeichnet, dass** es die folgenden Schritte umfasst:
- Dispergieren des DHA-Esters, insbesondere des im Anspruch 2 definierten DHA-Nikotinsäureesters oder des in den Ansprüchen 3 oder 4 definierten DHA-Panthenolesters, des Phospholipid-Derivats bzw. der Phospholipid-Derivate und des Polyoxyethylenfettsäureesters, insbesondere des Macrogol-15-Hydroxystearats, in einer wässrigen Lösung, die für eine parenterale Verabreichung vorgesehen ist, insbesondere einer 5%-igen Glukoselösung unter Rühren, insbesondere mit ungefähr 700 Umdrehungen/Minute, bis zum Erhalt einer homogenen, jedoch trüben Dispersion,
- Homogenisieren der erhaltenen Dispersion, beispielsweise mithilfe einer Rotor-/Statorturbine, insbesondere mit ungefähr 13.500 Umdrehungen/Minute, dann eines Hochdruckhomogenisators, insbesondere mit einem Druck zwischen 1300 und 1600 bar,
- Sterilisieren der erhaltenen kolloidalen Formulierung, beispielsweise durch Leiten durch einen 0,2-µm-Sterilisationsfilter.

11. Pharmazeutische Zusammensetzung nach einem der Ansprüche 1 bis 9, **dadurch gekennzeichnet, dass** sie dafür vorgesehen ist, intravenös, intraarteriell, intrakardial, subkutan, intradermal, intramuskulär, intraspinal, intrathekal, intraperitoneal, intraokulär, intraventrikulär, intraperikardial, intradural oder intraartikulär verabreicht zu werden.

12. Pharmazeutische Zusammensetzung nach einem der Ansprüche 1 bis 9 oder 11 zur Verwendung als Arzneimittel.

13. Pharmazeutische Zusammensetzung zur Verwendung als Arzneimittel nach Anspruch 12, **dadurch gekennzeichnet, dass** das besagte Arzneimittel zur Prävention und/oder zur Behandlung von Herz-Kreislauf-Erkrankungen, die vorteilhafterweise aus supraventrikulärer und/oder ventrikulärer Arrhythmie, Tachykardie und/oder Herzflimmern ausgewählt sind; zur Prävention und/oder zur Behandlung von Erkrankungen, die durch Störungen der elektrischen Leitung von Zellen des Herzmuskels dargestellt sind; zur Prävention und/oder zur Behandlung von mehreren Risikofaktoren von Herz-Kreislauf-Erkrankungen, die beispielsweise aus Hypertriglyceridämie, Hypercholesterinämie, Hyperlipidämien, Dyslipidämien, vorteilhafterweise gemischten Dyslipidämien, arterieller und venöser Thrombose, die von der Hyperaktivität des Blutgerinnungsfaktors II (Thrombin) und/oder Thrombozytenaggregation verursacht wird, und/oder arterieller Hypertonie ausgewählt sind; zur primären oder sekundären Prävention und/oder zur Behandlung von Herz-Kreislauf-Erkrankungen, die sich von supraventrikulärer und/oder ventrikulärer Arrhythmie, Tachykardie, Herzflimmern und/oder Störungen der elektrischen Leitung, die von einem Herzinfarkt induziert wurden, ableiten, vorteilhafter des plötzlichen Tods; und/oder zur Behandlung nach einem Infarkt vorgesehen ist.

## Claims

1. Pharmaceutical composition intended to be administered parenterally, containing submicronic docosahexanoic acid ester particles, dispersed in an aqueous phase by means of a mixture of at least two surfactants chosen from a) at least one polyoxyethylene fatty acid ester and b) at least one phospholipid.

2. Pharmaceutical composition according to claim 1, **characterized in that** the docosahexanoic acid ester is nicotinic ester.

3. Pharmaceutical composition according to claim 1, **characterized in that** the docosahexanoic acid ester is DHA pantothenic ester of the following formula A: 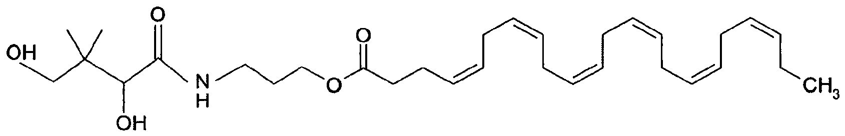 or one of its pharmaceutically acceptable salts, enantiomers, diastereosimers, or a mixture thereof, including racemic mixtures.

4. Pharmaceutical composition according to claim 3, **characterized in that** the docosahexanoic acid ester is DHA pantothenic ester of the following formula B:

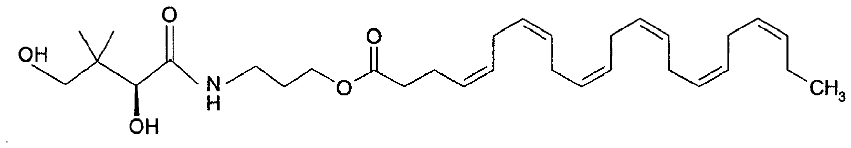

5. Pharmaceutical composition according to claim 1 to 4, **characterized in that** the polyoxyethylene fatty acid ester is macrogol-15 hydroxystearate.

6. Pharmaceutical composition according to any one of claims 1 to 5, **characterized in that** the phospholipid is chosen from lecithins of natural origin, such as, for example, soy or egg lecithins, phospholipids of natural origin, such as, for example, soy or egg phospholipids, synthetic phospholipids, or a mixture thereof.

7. Pharmaceutical composition according to claim 6, **characterized in that** the phospholipid derivative is a mixture of a neutral phospholipid and a negatively-charged phospholipid.

8. Pharmaceutical composition according to any one of claims 1 to 7, **characterized in that** the docosahexaenoic acid ester concentration is greater than or equal to 10 mg/ml, and advantageously greater than or equal to 30 mg/ml.

9. Pharmaceutical composition according to claim 1 to 8, **characterized in that** it is a dispersion of mixed micelles or vesicles or hybrids of micellar or vesicular structures.

10. Process for preparing a pharmaceutical composition according to any one of claims 1 to 9, **characterized in that** it includes the following steps:
- dispersing the DHA ester, in particular the DHA nicotinic ester defined in claim 2 or the DHA pantothenic ester defined in claims 3 or 4, the phospholipid derivative(s) and the polyoxyethylene fatty acid ester, in particular the macrogol 15 hydroxy stearate, in an aqueous solution intended for parenteral administration, in particular a 5% glucose solution, under agitation, in particular at around 700 rpm, until a homogeneous but turbid dispersion is obtained,
- homogenizing the dispersion obtained, for example by means of a rotor/stator turbine, in particular at around 13,500 rpm, then a high-pressure homogenizer, in particular at a pressure of between 1300 and 1600 bars,
- sterilizing the colloidal formulation obtained, for example, by passing through a 0.2 µm sterilizing filter.

11. Pharmaceutical composition according to any one of claims 1 to 9, **characterized in that** it is intended to be administered intravenously, intra-arterially, intracardially, subcutaneously, intradermally, intramuscularly, intrarachidially, intrathecally, intraperitoneally, intraocularly, intraventricularly, intrapericardially, intradurally, or intra-articularly.

12. Pharmaceutical composition according to any one of claims 1 to 9 or 11, for use as a drug.

13. Pharmaceutical composition for use as a drug according to claim 12, **characterized in that** said drug is intended for the prevention and/or treatment of cardiovascular diseases, advantageously chosen from supraventricular and/or ventricular arrhythmia, tachycardia and/or fibrillation, for example, atrial fibrillation; the prevention and/or treatment of diseases represented by electrical conduction defects of the myocardial cells; the prevention and/or treatment of multiple risk factors for cardiovascular diseases, for example chosen from hypertriglyceridemia, hypercholesterolemia, hyperlipidemia, dyslipidemia, advantageously, mixed dyslipidemia, arterial and venous thrombosis caused by factor II (thrombin) hyperactivity of blood coagulation and/or platelet aggregation, and/or arterial hypertension; the primary or secondary prevention and/or treatment of cardiovascular diseases resulting from supraventricular and/or ventricular arrhythmia, tachycardia, fibrillation and/or electrical conduction defects caused by myocardial infarction, advantageously sudden death; and/or post-infarction treatment.
